# EUROPEAN PATENT SPECIFICATION

(11) **EP 1 524 268 B1**
(45) Date of publication and mention of the grant of the patent: **17.01.2007**
(21) Application number: 04078092.6
(22) Date of filing: 12.02.1998
(51) Int. Cl.: C07D 303/46, C07D 303/34, C07C 253/30, A61K 31/275

(54) **Compounds and method for preparing sustituted 4-phenyl-4-cyanocyclohexanoic acids**
Verbindungen und Verfahren zur Herstellung substituierter 4-Phenyl-4-cyanocyclohexansäuren
Composés et procédé de préparation d'acides 4-phényle-4-cyanocyclohexanoiques substitués

(30) Priority: 12.02.1997 US 37608 P
(43) Date of publication of application: 20.04.2005
(62) Divisional of application: 02078056.5
(73) Proprietor: SMITHKLINE BEECHAM CORPORATION, Philadelphia, PA 19101 (US)
(72) Inventor: Allen, Andrew, Exton, PA 19341 (US); Diederich, Ann Marie, Downingtown, PA 19335 (US); Liu, Li, Collegeville, PA 19426 (US); Mendelson, Wilford, King of Prussia, PA 19406 (US)
(74) Representative: Povey, Alexander W.G.

(56) References cited:
- US-A- 5 552 438
- US-A- 5 602 157
- US-A- 5 602 173
- CHRISTENSEN S B ET AL: "1,4-Cyclohexanecarboxylates: Potent and Selective Inhibitors of Phosphodiesterase 4 for the Treatment of Asthma" JOURNAL OF MEDICINAL CHEMISTRY, AMERICAN CHEMICAL SOCIETY. WASHINGTON, US, vol. 41, 1998, pages 821-835, XP002173934 ISSN: 0022-2623

## Description

### Scope of the Invention

This invention covers a synthetic route for making 4-cyano-4-(3-cyclopentyloxy-4-methoxyphenyl)cyclohexanoic acid. This acid is selective for inhibiting the catalytic site in the phosphodiesterase isoenzyme denominated IV (PDE IV hereafter) and as such is useful in treating a number of diseases which can be moderated by affecting the PDE IV enzyme and its subtypes.

### Area of the Invention

Bronchial asthma is a complex, multifactorial disease characterized by reversible narrowing of the airway and hyper-reactivity of the respiratory tract to external stimuli.

Identification of novel therapeutic agents for asthma is made difficult by the fact that multiple mediators are responsible for the development of the disease. Thus, it seems unlikely that eliminating the effects of a single mediator will have a substantial effect on all three components of chronic asthma. An alternative to the "mediator approach" is to regulate the activity of the cells responsible for the pathophysiology of the disease.

One such way is by elevating levels of cAMP (adenosine cyclic 3',5'-monophosphate). Cyclic AMP has been shown to be a second messenger mediating the biologic responses to a wide range of hormones, neurotransmitters and drugs; [Krebs Endocrinology Proceedings of the 4th International Congress Excerpta Medica, 17-29, 1973]. When the appropriate agonist binds to specific cell surface receptors, adenylate cyclase is activated, which converts Mg⁺²-ATP to cAMP at an accelerated rate.

Cyclic AMP modulates the activity of most, if not all, of the cells that contribute to the pathophysiology of extrinsic (allergic) asthma. As such, an elevation of cAMP would produce beneficial effects including: 1) airway smooth muscle relaxation, 2) inhibition of mast cell mediator release, 3) suppression of neutrophil degranulation, 4) inhibition of basophil degranulation, and 5) inhibition of monocyte and macrophage activation. Hence, compounds that activate adenylate cyclase or inhibit phosphodiesterase should be effective in suppressing the inappropriate activation of airway smooth muscle and a wide variety of inflammatory cells. The principal cellular mechanism for the inactivation of cAMP is hydrolysis of the 3'-phosphodiester bond by one or more of a family of isozymes referred to as cyclic nucleotide phosphodiesterases (PDEs).

It has now been shown that a distinct cyclic nucleotide phosphodiesterase (PDE) isozyme, PDE IV, is responsible for CAMP breakdown in airway smooth muscle and inflammatory cells. [Torphy, "Phosphodiesterase Isozymes: Potential Targets for Novel Anti-asthmatic Agents" in New Drugs for Asthma, Barnes, cd. IBC Technical Services Ltd., 1989]. Research indicates that inhibition of this enzyme not only produces airway smooth muscle relaxation, but also suppresses degranulation of mast cells, basophils and neutrophils along with inhibiting the activation of monocytes and neutrophils. Moreover, the beneficial effects of PDE IV inhibitors are markedly potentiated when adenylate cyclase activity of target cells is elevated by appropriate hormones or autocoids, as would be the case *in vivo*. Thus PDE IV inhibitors would be effective in the asthmatic long, where levels of prostaglandin E₂ and prostacyclin (activators of adenylate cyclase) are elevated. Such compounds would offer a unique approach toward the pharmacotherapy of bronchial asthma and possess significant therapeutic advantages over agents currently on the market.

The process and intermediates of this invention provide a means for making certain 4-substituted-4-(3,4-disubstitutedphenyl)cyclohexanoic acids which are useful for treating asthma, and other diseases which can be moderated by affecting the PDE IV enzyme and its subtypes. The final products of particular interest are fully described in U.S. patent 5,552,483 issues 03 September 1996. The information and representations disclosed therein, in so far are that information and those representations are necessary to the understanding of this invention and in its practice, in total, are incorporated herein by reference.

US patents 5,552,438; 5,602,173; and 5,602,157 disclose certain inhibitors as mediators of the enzymatic or catalytic activity of phosphodiesterase IV

### Summary of the Invention

This invention relates to a process for enriching the *cis*-4-cyano-4-(3-cyclopentyloxy-4-methoxyphenyl)-*r*-cyclohexanecarboxylic acid form from a mixture of *cis* and *trans* isomers of the acid, which process comprises crystallizing from a hexane/ethyl acetate solution of *cis* and *trans* isomers the *cis* isomer essentially free of the *trans* isomer.

In one embodiment of the invention, the *cis*/*trans* mixture is dissolved in ethyl acetate and hexanes.

In another embodiment, the *cis*/*trans* mixture is dissolved in ethyl acetate and then hexanes are added.

In a further embodiment, the ethyl acetate solution of *cis*/*trans* isomers are heated to reflux, cooled and then hexanes added after which crystallization is carried out.

### Specific Embodiments of the Invention

The compound which is made by this process is a PDE IV inhibitors. It is useful for treating a number of diseases as described in U.S. patent 5,552,438 issued 3 September 1996.

A representative schematic of this process is set out in Scheme I. This graphical representation uses specific examples to illustrate the general methodology used in this invention.

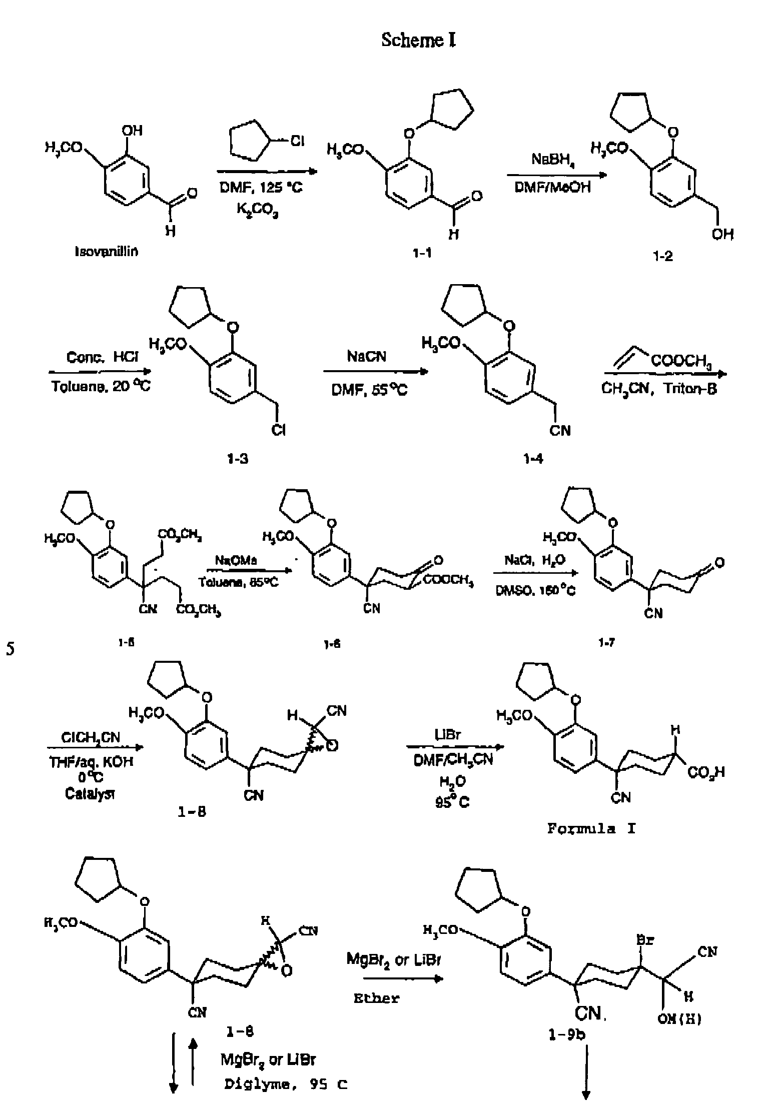

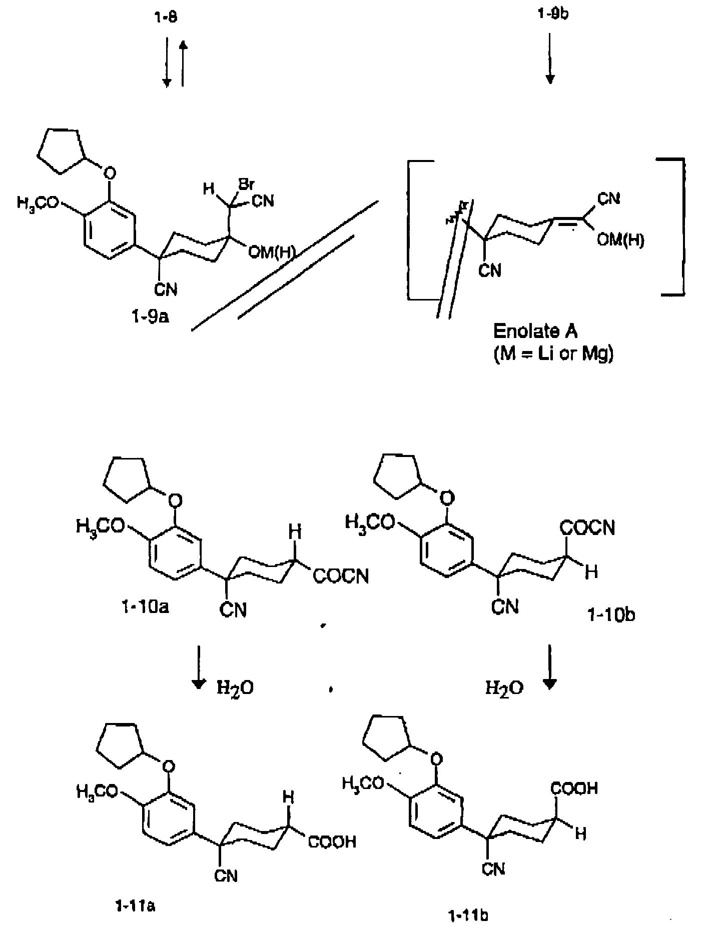

Referring to Scheme I, isovanillin, 3-hydroxy-4-methoxybenzaldehyde, is a readily available starting material. It can be alkylated with an R,X moiety (X = Cl, Br, and I) as represented by cyclopentyl chloride. The reaction vessel is first flushed with an inert gas, for example nitrogen. A polar solvent such as DMF is then added to the vessel, then the isovanillin, then the R,X adduct, and some base. About 2 equivalents of the R,X adduct versus the isovanillin are used. Likewise about 2 equivalents of base are used, again relative to the isovanillin. The base can be any inorganic base or a carbonate. Here it is illustrated by potassium carbonate. The vessel contents are heated to about 125° C for about 90 to 120 minutes in which time the reaction will have gone to completion. The vessel contents are cooled to ambient temperature, filtered to remove the inorganic salts, and washed with an alcohol such as methanol. This filtrate contains the aldehyde, labeled 1-1.

The aldehyde is then reduced to the alcohol using an inorganic reducing agent. To do this the filtrate from the foregoing reaction is treated with sodium borohydride and after workup affords the desired alcohol. 1-2 in 97% overall yield from isovanillin. This is achieved by cooling the filtrate to about 0° C after which a reducing agent, here sodium borohydride, is added. About 0.25 to 0.5 equivalents of this reducing agent is used. The temperature is keep at about 0° C during the addition of the reducing agent and for about 30 to 40 minutes thereafter. Then the temperature is allowed to rise to about room temperature after which about one-half an equivalent of HCl is added to the reaction vessel. The alcohol is then extracted into an organic solvent, toluene is illustrated, and washed with dilute sodium bicarbonate.

The top organic layer containing the alcohol is then treated with excess concentrated hydrochloric acid at ambient temperature to afford, after workup, the desired benzyl chloride 1-3. The chloride is isolated as a w/w solution in an amide solvent, DMF is illustrated, and treated with about a 50% molar excess of sodium cyanide at a mildly elevated temperature, here illustrated as 55° C. This affords the desired nitrile 1-4. The nitrile is isolated as a w/w solution in an appropriate solvent such as anhydrous acetonitrile and used directly in the next step.

The nitrile solution is charged with methyl acrylate. It is cooled to about -10° C, and slowly treated with a catalytic amount of Triton-B in the same solvent as used to dissolve the nitrile. The methyl acrylate is added in a 3 to 4-fold excess. The reaction is complete within 30 to 45 minutes after which the acrylate addition, the pimelate product, 1-5, is isolated as a w/w solution in toluene and treated with about 2 equivalents of sodium methoxide at about 75° C to give the β-kero-ester product, 1-6. The reaction solution is cooled and neutralized to pH 7 with mineral acid such as 6N hydrochloric acid. The solution is charged with dimethyl sulfoxide, sodium chloride, water, and heated, for example to about 150° C, to effect the decarboxylation to give 1-7. The ketone, 1-7, is isolated from the solvent system as an off-white solid.

The dicarbonitrile 1-8 is prepared from the ketone by treating the ketone with chloroacetonitrile in the presence of an inorganic base and a catalytic amount of benzyltriethylammonium chloride (BTEAC). The ketone is charged into a mixture of strong base (aqueous potassium hydroxide) and a water miscible solvent such as tetrahydrofuran. A slight excess of chloroacetonitrile is added at reduced temperature, about 0° C or thereabouts. The reaction is maintained at about that temperature for the duration of the reaction, usually about 1 hour. The product is isolated and usually it is crystalline.

The dicarbonitrile is converted to the cyclohexanecarboxylic acid using a Lewis acid catalyst; water is also needed to drive the reaction to the acid. Without water intermediates 1-10a and 1-10b may dimerize. This reaction is carried out by charging a vessel with solvents, in this instance exemplified by DMF, acetonitrile and water, and the Lewis acid (about 1.5 equivalents), LiBr is illustrated, sweeping the vessel with an inert gas, adding the dicarbonitrile IIa or IIb, or a mixture of IIa and IIb and heating the vessel and its contents to about 100° C for a number of hours, 8 hours being an example. The acid is isolated by conventional means.

It should be noted that this reaction, that is the conversion of the epoxide to the acid, involves several intermediates which do not need to be isolated. It has been found that treating the epoxide with LiBr yields intermediates 1-9a and 1-9b. Intermediate 1-9a is formed when LiBr is added to the reaction pot. But intermediate 1-9a converts back to the epoxide under the recited reaction conditions. Intermediate 1-9b is also formed but apparently reacts rapidly to form intermediates such as enolate A, 1-10a and 1-10b etc leading to product. So it appears 1-9a and 1-9b are formed, but that 1-9a converts back to the epoxide which ultimately forms 1-9b which is then converted to other intermediates enroute to forming the acids of 1-11a and 1-11b. Parenthetically the designation "OM(H)" in 1-9a and 1-9b means the metal salt of the alcohol or the alcohol per se, depending on the reaction conditions. Intermediate 1-9b is believed to convert to the acyl nitriles of formulas 1-10a and 1-10b via the proposed bracketed intermediate. The existence of the proposed bracketed intermediate (enolate) has not been fully confirmed. And while the acyl nitriles of 1-10a and 1-10b have not been directly observed, indirect evidence exists for these compounds by virtue of the fact the bis-condensation product dimer B was isolated and is analogous to reported compounds where a similar bis-condensate is the product of an acyl nitrile.

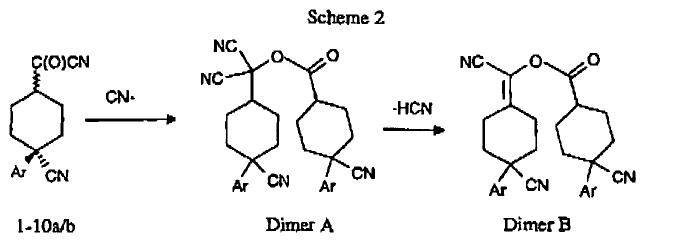

Dimers such as dimer A are known to form from the likes of acyl nitriles 1-10a/b in the presence of HCN (Thesing, J.; Witzel, D.; Brehm, A. *Angew Chem*., 1956, 68, 425; and Hunig, S.; Schaller, R. *Angew. Chem. Int. Ed. Engl*., 1982, 21,36).

And in addition, authentic samples of intermediates 1-10a and 1-10b were prepared and found to convert to acids 1-11a and 1-11b when exposed to water. The equitorial isomer 1-10a converted to the acid in an equitorial/axial ratio of about 98:2 while the axial isomer 1-10a isomerized to a perponderance of the equitorial isomer 1-11a (77:23). It is believed the axial acyl nitrile converts to the equitorial acyl nitrile via the proposed bracketed enolate intermediate.

The second, following reaction scheme illustrates preparing the acids of formula (I) from the bromoaldehyde of formula (IV).

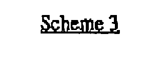

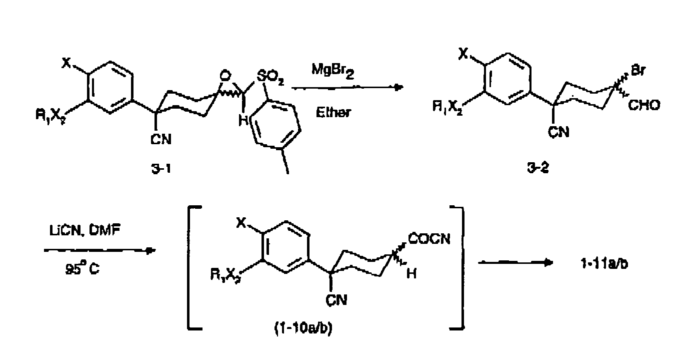

The following examples are provided to illustrate specifics of the invention, not to limit it. What is reserved to the inventors is set forth in the claims appended hereto.

### Specific Examples

### Reference Example 1

### Preparation of 3-cyclopentyloxy-4-methoxybenzaldehyde

A 12 liter round bottom flask equipped with an overhead stirrer, internal thermometer, and a reflux condensor equipped with a nitrogen inlet was flushed with nitrogen. The flask was charged with dimethylformamide (2.4 L), isovanillin (350 g, 2.3 mol, 1 equivalent), cyclopentyl chloride (481 g, 4.6 mol, 2.0 equivalent) and potassium carbonate (634 g, 4.6 mol, 2.0 equivalents). The vigorously stirred suspension was heated to 125 °C for two hours or until the disappearance of isovanillin. The reaction was cooled to 20 -30 °C and filtered to remove the inorganic salts. The filter cake was rinsed with methanol (1.0 L).

The clear, light-brown filtrate (DMF and methanol) containing the product, 3-cyclopentyloxy-4-methoxybenzaldehyde was used directly in the next step (100 % solution yield).

### Reference Example 2

### Preparation of 3-Cyclopentyloxy-4-methoxybenzyl alcohol.

A 12 liter round bottom flask equipped with an overhead stirrer, internal thermometer, and a reflux condensor equipped with a nitrogen inlet was flushed with nitrogen. The flask was charged with dimethylformamide (2.4 L), methanol (1.0 L), and 3-cyclopentyloxy-4-methoxybenzaldehyde (506 g, 2.3 mol, 1 equivalent). The contents of the flask were cooled to 0 to 5 °C followed by the addition of sodium borohydride (32.2 g, 0.85 mol, 0.37 equivalents). The reaction was maintained at 0 to 5 °C for 30 minutes, and warmed to 20 to 25 °C for an additional 2 hours or until the disappearance of the aldehyde. A solution of 6N hydrochloric acid (195 mL, 1.17 mol, 0.51 equivalents) was added over 20 minutes. The reaction was concentrated under reduced pressure, and cooled to 20 to 25 °C.

The flask was charged with deionized water (1.9 L) and toluene (1.9 L). The layers were separated, the organic layer was isolated, and washed twice with deionized water (2 x 800 mL). The product, 3-cyclopentyloxy-4-methoxybenzyl, alcohol was collected as a solution in toluene (97% solution yield) and used directly in the next step.

### Reference Example 3

### Preparation of 4-Chloromethyl-2-cyclopentyloxy-1-methoxybenzene.

A 12 liter round bottom flask equipped with an overhead stirrer, internal thermometer, and a reflux condensor equipped with a nitrogen inlet was flushed with nitrogen. The flask was charged with 3-cyclopentyloxy-4-methoxybenzyl alcohol (495 g, 2.2 mol. 1 equivalent) in a solution of toluene. To the vigorously stirred reaction at 22 °C was added concentrated hydrochloric acid (600 g, 2.75 equivalents). The reaction was maintained at 20 to 25 °C for 30 minutes. The top organic layer was isolated and the bottom acidic layer was discarded. To the top organic layer was charged a solution of 10% sodium bicarbonate (550 g, 0.65 mol, 0.36 equivalents) and t-butyl methyl ether (814 g). The contents of the flask were vigorously stirred, and allowed to settle. The product, 4-chloromethyl-2-cyclopentyloxy-1-methoxybenzene, was isolated as a solution in toluene and *t*-butyl methyl ether (96.8% solution yield). This was used directly in the next step.

### Reference Example 4

### Preparation of 4-Cyanomethyl-2-cyclopentyloxy-1-methoxybenzene.

A 12 liter round bottom flask equipped with an overhead stirrer, and a distillation apparatus was flushed with nitrogen. The flask was charged with 4-chloromethyl-2-cyclopentyloxy-1-methoxybenzene (519 g, 2.15 mol, 1.0 equivalents) in a solution of toluene and t-butyl methyl ether. The reaction was concentrated under reduce pressure to a residue. To the 12 liter flask was charged DMF (1.44 kg) and sodium cyanide (142 g. 2.9 mol, 1.35 equivalents). The reaction was heated to 55 °C for 6 hours or until deemed complete by the disappearance of the benzyl chloride. The reaction was concentrated under reduced pressure to a residue. To the flask was charged *t*-butyl methyl ether (2.30 kg) and deionized water (800 mL). The contents of the flask were vigorously stirred, and allowed to settle. The top organic layer was isolated, washed three times with deionized water (3 x 800 mL), and concentrated under atmospheric pressure to a residue. To the flask was added acetonitrile (1.26 kg) and the distillation was continued until an additional 400 mL of solvent was collected. The product, 4-cyanomethyl-2-cyclopentyloxy-1-methoxybenzene, was isolated as a solution in acetonitrile (92.2% yield). This was used directly in the next step.

### Reference Example 5

### Preparation of Dimethyl-4-cyano-4-(3-cyclopentyloxy-4-methoxy-phenyl)pimelare

A 12 liter round bottom flask equipped with an overhead stirrer, internal thermometer, and a reflux condensor equipped with a nitrogen inlet was flushed with nitrogen. The flask was charged with a solution of 4-cyanomethyl-2-cyclopentyloxy-1-methoxybenzene (460 g, 1.99 mol, 1.0 equivalent) in acetonitrile, and methyl acrylate (520 g, 6.0 mol, 3.0 equivalents). The contents of the flask was cooled to -10 °C. A pressure equalizing addition funnel was charged with acetonitrile (1.1 L) and benzyltrimethyl ammonium hydroxide (a 40% w/w solution in methanol, 25 g, 0.06 mol, 0.03 equivalents). The contents of the addition funnel was added to the flask. An exotherm was observed, and after stirring for 30 minutes the contents of the flask were cooled to 20 °C. The reaction was concentrated under reduced pressure to a residue. To the residue was added toluene (2.6 L). This solution of dimethyl-4-cyano-4-(3-cyclopentyloxy-4-methoxy-phenyl)pimelate (90% solution yield) was used directly in the next step.

### Reference Example 6

### Preparation of 4-Cyano-4-(3-cyclopentyloxy-4-methoxyphenyl)cyclohexan-1-one

A 12 liter round bottom flask equipped with an overhead stirrer, internal thermometer, and a reflux condensor equipped with a nitrogen inlet was flushed with nitrogen. The flask was charged with a solution of dimethyl-4-cyano-4-(3-cyclopentyloxy-4-methoxy-phenyl)pimelate (720 g, 1.78 mol, 1 equivalent) in toluene, and sodium methoxide (25 wt% in methanol, 545 g, 2.67 mol, 1.5 equivalents). The reaction was heated to 70 to 75 °C for 2 hours or until deemed complete by the disappearance of the pimelate. The reaction was cooled to 25 °C. A solution of 6N hydrochloric acid was added in order to adjust the pH to 6.8 - 7.2. The reaction was concentrated under vacuum to a residue. The flask was charged with dimethylsulfoxide (3.3 L), deionized water (250 mL) and sodium chloride (250 g).

The contents of the flask were heated to 145-155 °C, and held at this temperature for 2 hours. The reaction was cooled and concentrated under vacuum to a residue. To the residue was added water (1.9 L), ethyl acetate (1.25 L), and t-butyl methyl ether (620 mL). The solution was stirred and allowed to settle. The layers were separated, and the aqueous layer was re-extracted with ethyl acetate (1.25 L). The combined organic layers were washed twice with deionized water (2 X 2.5 L). The organic layer was isolated and concentrated under reduced pressure to a residue. To this residue was added isopropanol (1.66 L) and heated to produce a solution followed by the slow addition of hexanes (1.66 L). The suspension was cooled to 5 °C over 30 minutes, and held at 0 to 5 °C for two hours. The product was filtered and washed with 50-50 isopropanol-hexanes (840 mL) mixture at 0 °C, The product was dried to afford 4-cyano-4-(3-cyclopentyloxy-4-methoxyphenyl)cyclohexan-1-one (315 g, 56 % from the pimelate).

### Reference Example 7

### Preparation of cis-(+/-)-6-[3-(cyclopentyloxy)-4-methoxyphenyl]-1-oxobicyclo[2,5]octane-2,6-dicarbonitrile

A 5 liter round bottom flask equipped with an overhead stirrer, internal thermometer, and a nitrogen inlet was flushed with nitrogen. The flask was charged with 50% potassium hydroxide (220 g) and tetrahydrofuran (550 mL). While stirring at room temperature, benzyltriethylammonium chloride (8.1 g, 0.035 mol, 0.05 equivalent) was added. The solution was cooled to 0 °C. To a pressure equalizing addition funnel was charged a solution containing tetrahydrofuran (550 mL), 4-cyano-4-(3-cyclopentyloxy-4-methoxyphenyl) cyclohexan-1-one (230 g, 0.73 mol, 1.0 equivalent), and chloroacetonitrile (59 g, 0.78 mol. 1.07 equivalent) at room temperature. While stirring the flasks contents at 0 °C, the solution in the pressure addition funnel was added over 15 minutes. The temperature was maintained between 0 and 5 °C, and stirred for one hour. The reaction was warmed to 25 °C, diluted with water (900 mL), and ethyl acetate (900 mL). The solution was stirred and allowed to settle for 30 minutes. The layers were separated, the organic layer was isolated, and concentrated by vacuum distillation to a residue. Methanol (540 mL) was added and the solution was heated to 40 °C. While cooling to 20 °C over 90 minutes, hexanes (540 mL) was added. Cooling was continued, and the product began to crystallize at 10 °C. The suspension was then cooled to -5 °C and held at -5-0 °C for two hours. The product was filtered and washed with a 50-50 methanol-hexanes mixture (300 mL) at 0 °C. The product was dried to afford cis-(+/-)-6-[3-(cyclopentyloxy)-4-methoxyphenyl]-1-oxobicyclo[2.5]octane-2,6-dicarbonitrile (190 g. 73 %) as a white crystalline solid.

### Reference Example 8

### Preparation of 1-9a

In a stoppered 12 dram screw-top vial was added diglyme (5.92 g) and the epoxy nitrile (0.70 g, leq) of Example 7. This mixture was stirred with heating in an oil bath for 5 min. Then MgBr₂·6H₂O (0.906 g, 1.55 cq) was added. After 3 hr no starting material was detected. The reaction mixture was cooled, then mixed with 5% aqueous citric acid/ethyl acetate and the layers shaken and separated. The second extraction with ether/ethyl acetate gave some color extracted into organic layer, but the next extraction had no color. The organic fractions were combined and washed with water and brine and dried with MgSO₄. The product was crystallized from hexane; mp 151-152 °C.

Elemental analysis: C - 58.20, H - 5,82, Br - 18.44, N - 6.46; found C - 58.32, H - 5.73, Br - 18.48 , N - 6.34. The structure was confirmed by X-ray structure determination of a crystalline sample obtained from methyl alcohol.

### Reference Example 9

### Preparation of 1-9b

To Mg (0.189 g, 2.02 eq) (polished with mortar and pestle) in other was added 1,2-dibromoethane (1.55 g, 2.06 eq) in a small volume of ether to initiate the Grignard. When most of the magnesium was consumed and no more evolution of ethane was observed, the reaction was stirred for an additional 0.5 hr at room temperature after which was added the epoxide of Example 7 (1.41g; 1 eq) in a minimal amount of dry tetrahydrofuran at ambient temperature. After about 70 hr at room temperature there was obtained both the bromo cyanoalcohol (1-9b) and bromo cyanohydrin (1-9a) in a ratio of 6:1. The 1-9b product was isolated as an oil by prepartive HPLC. The structure was confirmed by carbon and proton NMR.

### Reference Example 10

### Preparation of Compound 3-1 - Epoxysulfone

To a 25ml round-bottom flask equipped with a magnetic stir bar and a rubber septum was charged 1.00 g of 4-cyano-4-(3-cyclopentyloxy-4-methoxyphenyl)cyclohexan-1-one, 0.70 g of chloromethyl *p*-tolylsulfome, and 7 ml of tetrahydrofuran. This was stirred, then 3 ml of 50% w/w aqueous NaOH and benzyltrimeth ch (0.05g) was added. This suspension was vigorously stirred for 2 hours at room temperature. The reaction solution was transferred to a separatory funnel to which was added 50 ml of ethyl acetate and acidified with 6N HCl. The organic layer was retained, washed 2X with deionized water, dried with MgSO₄ and filtered to remove the salts.

### Reference Example 11

### Preparation of Compound 3-2 - Bromoaldehyde

To magnesium (0.048g, 1.03 eq, 0.021 mol; polished with mortar and pestle) in ether was added 1,2-dibromoethane (0.40 g, 1.06 eq, 0.02 mol) under nitrogen. Two drops of iodine were added in ether to start the reaction, after which the reaction was heated gently. Once the Grignard had formed, the reaction flask was cooled to about 5 °C and the epoxysulfone of Example 10 (0.93 g. 1 eq, 0.002 mol) was added in ether/methylene chloride. The reaction was followed via TLC (conditions: silica gel with cyclohexane:toluene:acetonitrile:acetic acid 40:40:20:4). The reaction was stirred at 5° for 2 hours. The product was isolated by adding water and ether/TBME to the reaction mixture, and seperating the organic layers. These were washed with water and brine and dried over MgSO₄. Evaporation gave an oil which was flash chromatographed over 40 g of silica gel using a mixture of hexane and ethyl acetate (5-40% ethyl acetate). This gave a clear oil (0.49 g) containing about equal proportions of the equitorial and axial isomers as determined by proton NMR. Mass specrum showed a molecular ion at m/e 405 containing 1 bromine atom [C₂₀H₂₄BrNO₃].

### Example 12

### Preparation of c-4-cyano-4-(3-cyclopentyloxy-4-methoxyphenyl)-r-cyclohexanecarboxylic acid.

A 5 liter round bottom flask equipped with an overhead stirrer, internal thermometer, and a reflux condensor equipped with a nitrogen inlet was flushed with nitrogen. The flask was charged with dimethylformamide (580 g), acetonitrile (480 g), lithium bromide (72 g, 0.83 mol, 1.62 equivalents) and deionized water (20 g, 1,1 mol, 2.2 equivalents). The solution was stirred under nitrogen at 25 - 30 °C followed by the addition of cis-(+/-)-6-[3-(cyclopentyloxy)-4-methoxyphenyl]-1-oxobicyclo[2.5]octane-2,6-dicarbonitrile (180 g, 0.51 mol, 1.0 equivalent). The reactor was heated to 90 - 95 °C for 8 hours or until deemed complete by the disappearance of epoxy nitrite. The contents of the flask was cooled to 20 °C , followed by the addition of a sodium hydroxide solution (92 g of sodium hydroxide, 2.3 mol, 4.5 equivalents, dissolved in 200 mL of deionized water). The suspension was stirred at 20 °C for 30 minutes followed by the addition of sodium hypochlorite (600 mL, 0.46 mol, 0.9 equivalents). The contents of the flask were stirred for 90 minutes followed by the addition of t-butyl methyl ether (2.27 kg) and 6N HCl (644 mL, 3.86 mol, 7.5 equivalents). The bottom aqueous layer was back-extracted with *t*-butyl methyl ether (454 g), and the combined organic layer was washed four times with deionized water (4 X 800 mL). The organic layer was concentrated to a residue. To the flask was charged ethyl acetate (900 g) and heated to reflux. The contents of the flask was cooled to 50 °C followed by the addition of hexanes (672 g). The contents of the flask were cooled to 0 °C and held for I hour. The product was filtered and washed with cold ethyl acetate / hexanes (1/9, 175 g). The product was dried to afford *c*-4-cyano-4-(3-cyclopentyloxy-4-methoxyphenyl)-*r*-cyclohexanecarboxylic acid (125 g, 69 %) as an off-white powder.

### Reference Example 13

### Preparation of c-4-cyano-4-(3-cyclopentyloxy-4-methoxyphenyl)-r-cyclohexanecarboxylic acid chloride

In a 1 neck flask equipped for nitrogen flow was combined *c*-4-cyano-4-(3-cyclopentyloxy-4-methoxyphenyl)-*r*-cyclohexanecarboxylic acid (1.372g, 1 eq. 0.004 mol) and oxalyl chloride (4.568g, 9eq, 0.036 mol). One drop of dimethyl formamide was then added. This mixture was stirred at ambient temperature overnight. After evaporation under high vacuum this yielded the captioned product.

### Reference Example 14

### Preparation of Form 1-10a-4-cyano-4-(3-cyclopentyloxy-4-methoxyphenyl)-r-cyclohexane acyl nitrile

In a flask a sample of the acid chloride (0.217 g, 0.006 mol, 1 cq) prepared in Example 12 was dissolved in CDCl₃ (2.34 mL). To this was solution (cooled to 5°C) was added trimethylsilyl cyanide (0.07g, 1.3 eq, 0.008 mol.) and a catalytic amount of ZnI₂ (0.004g). This solution was refluxed overnight. This yielded 0.211g of the captioned product. IR: COCN, v 2220 cm⁻¹; C=O, v 1720cm⁻¹. The isomeric purity of 1-10a was determined by hydrolyzing the acyl nitrile in warm water, the product being essentially pure compound 1-11a.

### Reference Example 15

### Preparation of Form 1-10b of 4-cyano-4-(3-cyclopentyloxy-4-methoxyphenyl)-r-cyclohexane acyl nitrile

In an experiment analogous to Example 14, the axial carboxylic acid was converted to the acid chloride using oxalyl chloride and catalytic amount of dimethyl formamide. This acid chloride was converted directly to the corresponding acyl nitrile. 1-10b, the isomer of the compound prepared in Example 14. The isomeric purity was assayed by hydrolyzing the acyl nitrile by stirring it in warm water for 20 hours. Analytical HPLC determination showed that > 96% of the product had the form of 1-10b.

## Claims

1. A process for enriching the *cis*-4-cyano-4-(3-cyclopentyloxy-4-methoxyphenyl)-*r*-cyclohexanecarboxylic acid form from a mixture of *cis* and *trans* isomers of the acid, which process comprises crystallizing from a hexane/ethyl acetate solution of *cis* and *trans* isomers the *cis* isomer essentially free of the *trans* isomer.

2. The process of claim 1 wherein the *cis*/*trans* mixture is dissolved in ethyl acetate and hexanes.

3. The process of claim 2 where the *cis*/*trans* mixture is dissolved in ethyl acetate and then hexanes are added.

4. The process of claim 3 wherein the ethyl acetate solution of *cis*/*trans* isomers are heated to reflux, cooled and then hexanes added after which crystallization is carried out.

## Patentansprüche

1. Verfahren zum Anreichern der cis-4-Cyano-4-(3-cyclopentyloxy-4-methoxyphenyl)-r-cyclohexancarbonsäure-Form aus einer Mischung der cis-und trans-Isomere der Säure, wobei das Verfahren das Kristallisieren des cis-Isomers, das im wesentlichen frei vom trans-Isomer ist, aus einer Hexan/Ethylacetat-Lösung der cis- und trans-Isomere umfaßt.

2. Verfahren gemäß Anspruch 1, worin die cis/trans-Mischung in Ethylacetat und Hexanen gelöst wird.

3. Verfahren gemäß Anspruch 2, worin die cis/trans-Mischung in Ethylacetat gelöst wird und dann Hexane hinzugegeben werden.

4. Verfahren gemäß Anspruch 3, worin die Ethylacetat-Lösung der cis/trans-Isomere zum Rückfluß erwärmt wird, abgekühlt wird und dann Hexane hinzugegeben werden, worauf die Kristallisation durchgeführt wird.

## Revendications

1. Procédé d'enrichissement de la forme *cis-* de l'acide 4-cyano-4-(3-cyclopentyloxy-4-méthoxyphényl)-r-cyclohexanecarboxylique à partir d'un mélange d'isomères *cis* et *trans* de l'acide, lequel procédé comprend la cristallisation à partir d'une solution d'isomères *cis* et *trans* dans le mélange hexane/acétate d'éthyle, de l'isomère *cis* essentiellement exempt d'isomère *trans*.

2. Procédé selon la revendication 1, dans lequel le mélange *cis*/*trans* est dissous dans l'acétate d'éthyle et les hexanes.

3. Procédé selon la revendication 2, dans lequel le mélange *cis*/*trans* est dissous dans l'acétate d'éthyle et les hexanes y sont ensuite ajoutées.

4. Procédé selon la revendication 3, dans lequel la solution des isomères *cis*/*trans* dans l'acétate d'éthyle est chauffée au reflux, refroidie, et ensuite additionnée d'hexanes, après quoi la cristallisation est effectuée.
